# EUROPÄISCHE PATENTSCHRIFT

(11) **EP 0 777 500 B1**
(45) Veröffentlichungstag und Bekanntmachung des Hinweises auf die Patenterteilung: **02.12.1998**
(21) Anmeldenummer: 95925811.2
(22) Anmeldetag: 03.07.1995
(51) Int. Cl.: A61K 49/00

(54) **ORALES ECHOKONTRASTMITTEL FÜR ULTRASCHALLDIAGNOSTIK**
ORAL CONTRAST AGENT FOR ULTRASOUND DIAGNOSTIC PROCEDURES
SUBSTANCE DE CONTRASTE ADMINISTREE PAR VOIE ORALE POUR DIAGNOSTIC ECHOGRAPHIQUE PAR ULTRASONS

(30) Priorität: 09.07.1994 DE 4424233
(43) Veröffentlichungstag der Anmeldung: 11.06.1997
(73) Patentinhaber: Gieselmann, Thomas, 56288 Kastellaun (DE)
(72) Erfinder: Gieselmann, Thomas, 56288 Kastellaun (DE)
(74) Vertreter: Müller-Gerbes, Margot, Dipl.-Ing.
(86) Internationale Anmeldenummer: EP9502569
(87) Internationale Veröffentlichungsnummer: WO9601654

(56) Entgegenhaltungen:
- EP-A- 0 077 752
- WO-A-91/18612
- CHEMICAL AND PHARMACEUTICAL BULLETIN, Bd. 30, Nr. 10, 1982 TOKYO JP, Seiten 3701-3710, KOZO TAKAYAMA ET AL. 'DISSOLUTION BEHAVIOUR OF FLUFENAMIC ACID DISPERSED IN CROSS-LINKED INSOLUBLE POLYVINYLPYROLLIDONE: EFFECT OF WATER-SOLUBLE POLYMERS ADDED AS THE THIRD COMPONENT.'

## Beschreibung

Die Erfindung befaßt sich mit einem oralen Echokontrastmittel für Ultraschalldiagnostik zur Darstellung des gesamten Magen-Darm-Traktes. Ein Echokontrastmittel hat die folgenden Anforderungen zu erfüllen:

Es darf nicht toxisch sein, es darf die Darmperistaltik nicht beeinflussen, es darf nicht resorbiert werden, und es darf keine Schallschatten verursachen, die die Diagnose erschweren würden. Des weiteren muß das Echokontrastmittel den Darm dehnen und den Darm homogen ausfüllen.

Als orales Echokontrastmittel für die Ultraschalldiagnostik wurden bisher überwiegend Wasser, Fruchtsäfte, Milch als Hilfsmittel eingesetzt. Die Ergebnisse der damit durchgeführten Ultraschalluntersuchungen sind dennoch unbefriedigend. Hinzu kommt, daß Patienten zwischen einem und zwei Litern Flüssigkeit zu sich nehmen müssen.

Neuerdings ist auch versucht worden, Zellulosefasern oder andere lufthaltige Hohlkörper oder Schichtsilikate als Echokontrastmittel einzusetzen, die jedoch sich insofern als nachteilig erwiesen haben, als Schallschatten auftreten, die besonders in tieferen Schichten eine Diagnose unmöglich machen.

Der Erfindung liegt die Aufgabe zugrunde, ein orales Echokontrastmittel für die Ultraschalldiagnostik für die Humanmedizin zur Untersuchung des Magen-Darm-Traktes vorzuschlagen, das eine detailreiche Darstellung des untersuchten Gebietes ermöglicht und das Auftreten von störenden Schallschatten unterbindet.

Überraschenderweise wurde gefunden, daß ein orales Echokontrastmittel, enthaltend auf ein Liter Wasser die folgenden Komponenten:
A) 0,5 bis 80 g, vorzugsweise 1 bis 40 g mindestens eines Verdickungsmittels,
B) 4 bis 100 g, vorzugsweise 10 bis 60 g Polyvinylpolypyrrolidon (PVPP),
C) 0 bis 40 g, vorzugsweise 8 bis 20 g Mannit,
D) 0 bis 2 g, vorzugsweise 0,5 bis 1,5 g Aromastoffe,
E) 0 bis 50 g, vorzugsweise 10 bis 30 g eines Lösungsmittels für eine der Komponenten A,
F) 0 bis 5 g, vorzugsweise 0,1 bis 3 g nichtionisches Tensid,
diese Anforderungen erfüllt. Hierbei ist es insbesondere die Kombination aus mindestens einem Verdickungsmittel und einem quervernetzten unlöslichen Polyvinylpolypyrrolidon, das auch als Crosspovidon bezeichnet wird, die eine Kontrastanhebung bewirkt, die zu dem gewünschten Diagnoseerfolg führt.

Insbesondere wird mit dem erfindungsgemäßen oralen Echokontrastmittel eine scharfe Demarkierung der Darm-Mukosa erreicht und damit eine sehr gute Möglichkeit, andere Organe durch das so entstandene Schallfenster untersuchen zu können.

Alle für das erfindungsgemäße orale Echokontrastmittel eingesetzten Komponenten sind unter dem Gesichtspunkt auszusuchen, daß sie nicht toxisch sind, die Darm-Peristaltik nicht beeinflussen, nicht resorbiert werden und keine Schallschatten verursachen, andererseits den Darm dehnen und diesen homogen ausfüllen.

Als Verdickungsmittel kommen insbesondere organische natürliche Verdickungsmittel und/oder organische abgewandelte Naturstoffe und/oder vollsynthetische organische Stoffe in Frage.

Als organische natürliche Verdickungsmittel sind Agar-Agar, Tragant, Carrageen, Alginate, Gummi arabicum, Pektine, Polyosen, Guar-Mehl, Stärke, Xanthan, Dextrine, Gelatine und/oder Casein bevorzugt. Als Verdickungsmittel auf Basis organisch abgewandelter Naturstoffe kommen insbesondere Carboxymethylcellulose und/oder Celluloseether in Frage.

Die organischen natürlichen Verdickungsmittel und die auf Basis organisch abgewandelter Naturstoffe erhaltenen Verdickungsmittel sind überwiegend gut wasserlöslich.

Als vollsynthetisches organisches Verdickungsmittel kommen auch mittel- und hochmolekulare Polyvinylpyrrolidone, auch Povidone genannt, in Frage, die sehr gut wasserlöslich sind.

Um die Darm-Mukosa besser benetzen zu können, wird erfindungsgemäß vorgeschlagen, dem Echokontrastmittel ein geeignetes pharmazeutisch bzw. lebensmittelrechtlich zugelassenes Tensid, bevorzugt ein nichtionisches Tensid zuzusetzen. Hierfür kommt beispielsweise ein als nichtionisches Tensid wirksames Poloxamer in Frage. Es ist auch möglich, die sogenannten Zuckertenside einzusetzen. Auch andere lebensmittelrechtlich zugelassene Tenside können für die gewünschten Zweck benutzt werden.

Dem erfindungsgemäßen Echokontrastmittel kann des weiteren Mannit zugesetzt werden, das neben der Süßstoffwirkung auch eine leicht abführende Wirkung aufweist. Auch das Poloxamer, wenn es als Tensid eingesetzt wird, um die Darm-Mukosa besser zu benetzen, hat eine leicht abführende Wirkung.

Eine solche abführende Wirkung ist jedoch für das Wohlbefinden des mit dem erfindungsgemäßen Echokontrastmittel behandelten Patienten ebenfalls wünschenswert.

Dem erfindungsgemäßen Echokontrastmittel können des weiteren Geschmackskorrigenzien, insbesondere Aromastoffe und eventuell auch ein Konservierungsmittel zugegeben werden. Hierfür kommen ebenfalls die lebensmittelrechtlich zugelassenen Aromastoffe und Konservierungsmittel in Frage.

Die bevorzugten Mengen der einzelnen Komponenten sind im Anspruch 1 angegeben, und zwar in der auf ein Liter Wasser berechneten Menge, womit ein durch den Patienten gut einnehmbares pastöses Echokontrastmittel erhalten wird.

Um eine gleichmäßige Verteilung der einzelnen Komponenten in dem Echokontrastmittel zu erhalten, kann es zweckmäßig sein, eines der Verdickungsmittel separat in einem Lösungsmittel zu verteilen und dann in dieser Vorverteilung dem Wasser zuzugeben. Als ein solches zusätzliches Lösungsmittel kommt beispielsweise Propylenglykol in geringen Mengen zum Einsatz. Auch andere geeignete und lebensmittelrechtlich zugelassene Lösungsmittel können hier eingesetzt werden.

Eine bevorzugte Zusammensetzung eines erfindungsgemäßen Echokontrastmittels enthält auf ein Liter Wasser als Komponente
A) 2 bis 10 g Xanthan und 2 bis 3 g Povidone, als
B) 20 bis 60 g PVPP, als
C) 5 bis 30 g Mannit, als
D) 0,1 bis 1,2 g Aromastoffe, als
E) 10 bis 30 g Propylenglykol und als
F) 0 bis 5 g Poloxamer.

Humanversuche mit gemäß der Erfindung zusammengesetztem Echokontrastmittel haben die gute Verträglichkeit und ihre überraschenden echogenen Eigenschaften erwiesen. Orale Echokontrastmittel gemäß der Erfindung bewirken eine gleichmäßige Schallabsorption bzw. Schallreflexion bei der Ultraschalldiagnostik bei gleichzeitiger Verdrängung störender Luft. Eine detailreiche Darstellung des gesamten Magen-Darm-Traktes wurde erreicht. Des weiteren kann die Wanddicke von Magen und Darm gut dargestellt werden.

Die Erfindung wird nachfolgend anhand zweier beispielhafter Zusammensetzungen eines oralen Echokontrastmittels dargestellt.

### Beispiel 1:

- Komponente A:: Xanthan - Gum (Handelsbezeichnung Keltrol F ® der Firma.Kelco) 4 g
Povidone (Handeslbezeichnung Kollidon 17 der Firma BASF) 20 g
- Komponente B:: PVPP (Handelsbezeichnung Polyplasdone XL ® der Firma GAF) 36 g
- Komponente C:: Mannit 10 g
- Komponente D:: Aromastoff 1,0 g
- Komponente E:: Propylenglykol 20 g
- Wasser:: 1 Liter (1000 g)

Zur Herstellung des Echokontrastmittels wurde das Mannit, das Povidon und der Aromastoff in dem Wasser gelöst. Danach wurde das PVPP suspendiert.

In einem separaten Gefäß wird das Xanthan in Propylenglykol homogen verteilt und unter Rühren der vorgenannten wässrigen Flüssigkeit zugegeben.

Nach dem Quellen des Xanthan ist die Zubereitung gebrauchsfertig als Echokontrastmittel.

### Beispiel 2:

- Komponente A:: Xanthan, wie Beispiel 1, 3,0 g
Povidon, wie Beispiel 1, 3,0 g
- Komponente B:: PVPP, gemäß Beispiel 1, 40,0 g
- Komponente C:: Mannit 15,0 g
- Komponente D:: Aromastoff 1,0 g
- Komponente E:: Propylenglykol 20,0 g
- Komponente F:: Poloxamer 188 2,0 g
- Wasser:: 1 Liter

Die Herstellung der Zubereitung erfolgt analog wie in Beispiel 1 beschrieben. Die Komponente F wird zusammen mit dem Mannit und dem Povidon und den Aromastoffen in dem Wasser gelöst.

## Patentansprüche

1. Orales Echokontrastmittel für Ultraschall-Diagnostik, enthaltend auf ein Liter Wasser die folgenden Komponenten:
A) 0,5 bis 80 g, vorzugsweise 1 bis 40 g mindestens eines Verdickungsmittels,
B) 4 bis 100 g, vorzugsweise 10 bis 60 g Polyvinylpolypyrrolidon (PVPP),
C) 0 bis 40 g, vorzugsweise 8 bis 20 g Mannit,
D) 0 bis 2 g, vorzugsweise 0,5 bis 1,5 g Aromastoffe,
E) 0 bis 50 g, vorzugsweise 10 bis 30 g eines Lösungsmittels für eine der Komponenten A,
F) 0 bis 5 g, vorzugsweise 0,1 bis 3 g nichtionisches Tensid.

2. Echokontrastmittel nach Anspruch 1,
**dadurch gekennzeichnet**, daß organische natürliche Verdickungsmittel und/oder organische abgewandelte Naturstoffe und/oder vollsynthetische organische Stoffe als Verdickungsmittel eingesetzt sind.

3. Echokontrastmittel nach Anspruch 1 oder 2,
**dadurch gekennzeichnet**, daß als organische natürliche Verdickungsmittel Agar-Agar, Tragant, Carrageen, Alginate, Gummi arabicum, Pektine, Polyosen, Guar-Mehl, Stärke, Xanthan, Dextrine, Gelatine und/oder Casein eingesetzt sind.

4. Echokontrastmittel nach einem der Ansprüche 1 bis 3,
**dadurch gekennzeichnet**, daß als Verdickungsmittel auf Basis organisch abgewandelter Naturstoffe Carboxymethylcellulose und/oder Celluloseether eingesetzt sind.

5. Echokontrastmittel nach einem der Ansprüche 1 bis 4,
**dadurch gekennzeichnet**, daß als vollsynthetisches Verdickungsmittel mittel- oder hochmolekulare Povidone (Polyvinylpyrrolidone) eingesetzt sind.

6. Echokontrastmittel nach einem der Ansprüche 1 bis 5,
**dadurch gekennzeichnet**, daß mindestens eines der eingesetzten Verdickungsmittel gut in Wasser löslich ist.

7. Echokontrastmittel nach einem der Ansprüche 1 bis 6,
**dadurch gekennzeichnet**, daß als Komponente F ein lebensmittelrechtlich zugelassenes als nichtionisches Tensid wirksames Poloxamer eingesetzt ist.

8. Echokontrastmittel nach einem der Ansprüche 1 bis 7,
**dadurch gekennzeichnet**, daß als Lösungsmittel Propylenglykol eingesetzt ist.

9. Echokontrastmittel nach einem der Ansprüche 1 bis 8,
**gekennzeichnet durch** eine Zusammensetzung, enthaltend auf ein Liter Wasser als Komponente
A) 2 bis 10 g Xanthan und 2 bis 3 g Povidone, als
B) 20 bis 60 g PVPP, als
C) 5 bis 30 g Mannit, als
D) 0,1 bis 1,2 g Aromastoffe, als
E) 10 bis 30 g Propylenglykol und als
F) 0 bis 5 g Poloxamer.

## Claims

1. Oral echo contrast medium for ultrasonic diagnosis, containing the following components in one liter of water:
A) 0.5 to 80 g, preferably 1 to 40 g of at least one thickening agent;
B) 4 to 100 g, preferably 10 to 60 g of polyvinylpolypyrrolidone (PVPP);
C) 0 to 40 g, preferably 8 to 20 g of mannite;
D) 0 to 2 g, preferably 0.5 to 1.5 g of aromatic substance;
E) 0 to 50 g, preferably 10 to 30 g of a solvent for one of components A;
F) 0 to 5 g, preferably 0.1 to 3 g of non-ionic surfactant.

2. Echo contrast medium according to the Claim 1, characterized in that organic natural thickening agents and/or organically converted natural substances and/or fully synthetic organic substances are used as thickening agents.

3. Echo contrast medium according to Claim 1 or 2, characterized in that agar-agar, gum tragacanth, carrageenan, alginates, gum arabic, pectins, polyoses, guar powder, starch, xanthene, dextrins, gelatins, and/or casein are used as organic natural thickening agents.

4. Echo contrast medium according to one of Claims 1 to 3, characterized in that carboxymethylcellulose and/or cellulose ether are used as thickening agents based on organically converted natural materials.

5. Echo contrast medium according to one of Claims 1 to 4, characterized in that medium or high molecular weight povidones (polyvinyl pyrrolidones) are used as fully synthetic thickening agents.

6. Echo contrast medium according to one of Claims 1 to 5, characterized in that at least one of the thickening agents used is readily soluble in water.

7. Echo contrast medium according to one of Claims 1 to 6, characterized in that a poloxamer that has been approved by legislation governing food and is a non-ionic surfactant is used as component F.

8. Echo contrast medium according to one of Claims 1 to 7, characterized in that propylene glycol is used as the solvent.

9. Echo contrast medium according to one of Claims 1 to 8, characterized by a composition that contains the following as components in one liter of water:
A) 2 to 10 g xanthene and 2 to 3 g povidone,
B) 20 to 60 g PVPP,
C) 5 to 30 g mannite,
D) 0.1 to 1.2 g aromatic substance,
E) 10 to 30 g propylene glycol, and
F) 0 to 5 g poloxamer.

## Revendications

1. Substance de contraste d'écho administrée par voie orale pour le diagnostic ultrasonographique, contenant pour un litre d'eau les composants suivants:
A) 0,5 à 80 g, de préférence 1 à 40 g d'au moins un épaississant,
B) 4 à 100 g, de préférence 10 à 60 g de polyvinylpolypyrrolidone (PVPP),
C) 0 à 40 g, de préférence 8 à 20 g de mannitol,
D) 0 à 2 g, de préférence 0,5 à 1,5 g de substances aromatisantes,
E) 0 à 50 g, de préférence 10 à 30 g d'un solvant pour un des composants A,
F) 0 à 5 g, de préférence 0,1 à 3 g de tensioactif non ionique.

2. Substance de contraste d'écho selon la revendication 1, **caractérisée en ce que** l'on met en oeuvre comme épaississants, des épaississants organiques naturels et/ou des substances naturelles modifiées organiques et/ou des substances organiques entièrement synthétiques.

3. Substance de contraste d'écho selon la revendication 1 ou 2, **caractérisée en ce que** l'on met en oeuvre comme épaississants organiques naturels, la gélose, la gomme adragante, le carragaheen, les alginates, la gomme arabique, les pectines, les polyoses, la farine de guar, l'amidon, la xanthane, les dextrines, les gélatines et/ou la caséine.

4. Substance de contraste d'écho selon une des revendications 1 à 3, **caractérisée en ce que** l'on met en oeuvre comme épaississant à base de substances naturelles modifiées organiques, la carboxyméthylcellulose et/ou l'éther cellulosique.

5. Substance de contraste d'écho selon une des revendications 1 à 4, **caractérisée en ce que** l'on met en oeuvre comme épaississant entièrement synthétique, des povidones de poids moléculaire moyen ou élevé (polyvinylpyrrolidones).

6. Substance de contraste d'écho selon une des revendications 1 à 5, **caractérisée en ce qu**'au moins un des épaississants mis en oeuvre est bien soluble dans l'eau.

7. Substance de contraste d'écho selon une des revendications 1 à 6, **caractérisée en ce que** l'on met en oeuvre comme composant F, un poloxamère, actif comme surfactif non ionique, autorisé par la législation sur les produits alimentaires.

8. Substance de contraste d'écho selon une des revendications 1 à 7, **caractérisée en ce que** l'on met en oeuvre comme solvant, du propylèneglycol.

9. Substance de contraste d'écho selon une des revendications 1 à 8, **caractérisée** par une composition, contenant pour un litre d'eau, comme composants
A) 2 à 10 g de xanthane et 2 à 3 g de povidone,
B) 20 à 60 g de PVPP,
C) 5 à 30 g de mannitol,
D) 0,1 à 1,2 g de substances aromatiques,
E) 10 à 30 g de propylèneglycol et
F) 0 à 5 g de poloxamère.
